# FASCICULE DE BREVET EUROPEEN

(11) **EP 2 863 999 B1**
(45) Date de publication et mention de la délivrance du brevet: **02.08.2017**
(21) Numéro de dépôt: 13737337.9
(22) Date de dépôt: 20.06.2013
(51) Int. Cl.: A61Q 5/02, A61Q 5/12, A61K 8/97, A61Q 5/00, A61K 8/25, A61K 8/02, A61K 8/73, A61Q 19/00, A61Q 1/00

(54) **COMPOSITION COSMÉTIQUE DES PARTICULES D'AÉROGEL DE SILICE HYDROPHOBE ET UN POLYMÈRE À MOTIF SUCRE**
KOSMETISCHE ZUSAMMENSETZUNG AUS HYDROPHOBEN SILIZIUM AEROGEL PARTIKELN UND EINEM POLYMER MIT EINER ZUCKEREINHEIT
COSMETIC COMPOSITION OF HYDROPHOBIC SILICA AEROGEL PARTICLES AND A POLYMER COMPRISING A SUGAR UNIT

(30) Priorité: 21.06.2012 FR 1255815; 16.11.2012 US 201261727144 P
(43) Date de publication de la demande: 29.04.2015
(73) Titulaire: L'Oréal, 75008 Paris (FR)
(72) Inventeur: KHENNICHE, Samira, F-92110 Clichy (FR); PLOS, Grégory, F-75018 Paris (FR)
(74) Mandataire: Dodin, Catherine
(86) Numéro de dépôt international: PCT/FR2013/051437
(87) Numéro de publication internationale: WO 2013/190239

(56) Documents cités:
- WO-A2-2009/059869
- FR-A1- 2 150 914
- FR-A1- 2 913 334
- FR-A1- 2 929 113
- US-A- 5 702 690
- DOW CORNING: "Dow Corning VM-2270 Aerogel Fine particles", INTERNET CITATION, avril 2009 (2009-04), pages 1-5, XP002650585, Extrait de l'Internet: URL:http://www2.dowcorning.com/DataFiles/0 90007c88020e235.pdf [extrait le 2011-07-15]

## Description

La présente invention concerne une composition cosmétique, notamment capillaire, comprenant l'association de particules d'aérogel de silice hydrophobe et de polymères épaississants particuliers, ainsi qu'un procédé de mise en oeuvre de ladite composition.

Les chevelures ont tendance à perdre certaines de leurs qualités sous l'action de facteurs tels que le regraissage naturel, la sueur, l'élimination de squames, la pollution, l'humidité et autres facteurs. L'aspect visuel ou le toucher des cheveux peut ainsi être dégradé. Le regraissage, par exemple dû à la pollution, alourdit les cheveux qui ont alors tendance à se mettre en paquets. Les cheveux peuvent être difficiles à coiffer en plus d'avoir par exemple une brillance grasse ou un toucher ciré désagréable. Pour lutter contre ces désagréments, on peut utiliser des compositions détergentes, par exemple des shampoings, afin d'éliminer les salissures (sébum, sueur, pollution, etc.) ou les pellicules, et détendre les cheveux. La chevelure est ensuite rincée puis séchée. Les shampooings doivent être renouvelés régulièrement, par exemple au bout de quelques jours voire au bout de quelques heures. Toutefois, les shampooings, à base de quantités importantes de tensioactifs, peuvent générer des désagréments tels que des picotements sur le cuir chevelu ou dans les yeux.
Les compositions de shampoing ou de lavage de la peau peuvent également associer aux tensioactifs, des absorbeurs de sébum pour permettre de prolonger dans le temps la perception de propreté des cheveux ou de la peau. Toutefois, on a constaté que l'efficacité de ces produits est encore insuffisante par rapport aux attentes des consommateurs. En effet, ils ne permettent pas de réduire de manière significative la fréquence de lavage des cheveux notamment, fréquence qui peut différer selon le pays ou même de la région concernée et qui peut aller de un à deux shampooings par jour à un ou deux shampooings par semaine.

A ce jour, aucune composition cosmétique d'hygiène, notamment capillaire, ne permet de ralentir de manière significative le regraissage, notamment des cheveux.

La présente invention a pour but de proposer des compositions cosmétiques palliant ces inconvénients.
Les compositions selon l'invention permettent de conserver une perception de cheveux propres pendant un temps plus long qu'avec un shampoing usuel; à titre d'exemple, cette perception de cheveux propres peut être d'une semaine pour les personnes se lavant habituellement les cheveux 2 à 3 fois par semaine, et peut être d'au moins trois jours pour les personnes se lavant habituellement les cheveux tous les jours.
Par ailleurs, les compositions selon l'invention permettent d'obtenir des performances de nettoyage au moins identiques à celles d'un shampooing standard, notamment un très bon pouvoir de détergence.

L'invention a donc pour objet une composition cosmétique comprenant des particules d'aérogel de silice hydrophobe et au moins un polymère à motif(s) sucre(s).

On a constaté que l'utilisation des compositions selon l'invention permet de réduire de manière significative le regraissage des cheveux et/ou du cuir chevelu, et permet ainsi de réduire la fréquence de lavage.
En outre, la composition selon l'invention permet d'obtenir de bonnes propriétés de conditionnement des cheveux, notamment en terme de douceur, souplesse, lissage et démêlage, tout en ayant une répartition et un étalement sur la chevelure améliorés.

Dans la présente description, l'expression "au moins un" est équivalente à l'expression "un ou plusieurs" et peut y être substituée.
Dans la présente description, l'expression "compris entre" est équivalente à l'expression "allant de " et peut y être substituée.

### Particules d'aérogel de silice hydrophobe

La composition selon l'invention comprend donc des particules d'aérogel de silice hydrophobe.

Les aérogels sont des matériaux poreux ultra légers, dont les premiers ont été réalisés par Kristler en 1932.
Ils sont généralement synthétisés par un procédé sol-gel en milieu liquide puis séchés par extraction d'un fluide supercritique. Le fluide supercritique le plus communément utilisé est le CO₂ supercritique. Ce type de séchage permet d'éviter la contraction des pores et du matériau. D'autres types de séchage permettent également d'obtenir des matériaux poreux à partir de gel, à savoir par exemple (i) le séchage par cryodessiccation, consistant à solidifier à faible température le gel puis à sublimer le solvant et (ii) le séchage par évaporation. Les matériaux ainsi obtenus sont appelés respectivement cryogels et xérogels. Le procédé sol-gel et les différents séchages sont décrits en détail dans Brinker CJ., and Scherer G.W., Sol-Gel Science: New York: Academic Press, 1990.

Par "silice hydrophobe", on entend toute silice dont la surface est traitée par des agents de silylation, par exemple par des silanes halogénés tels que des alkylchlorosilanes; des siloxanes en particulier des diméthylsiloxanes tels que l'hexaméthyldisiloxane; ou des silazanes, de manière à fonctionnaliser les groupements OH par des groupements silyles Si-Rn, par exemple des groupements triméthylsilyles.

De préférence, les particules d'aérogel de silice hydrophobe susceptibles d'être utilisées dans la présente invention présentent avantageusement une surface spécifique par unité de masse (SM) allant de 500 à 1500 m²/g, de préférence de 600 à 1200 m²/g et mieux de 600 à 800 m²/g.
De préférence, les particules d'aérogel de silice hydrophobe susceptibles d'être utilisées dans la présente invention présentent avantageusement une capacité d'absorption d'huile mesurée au WET POINT allant de 5 à 18 ml/g de particules, de préférence de 6 à 15 ml/g et mieux de 8 à 12 ml/g.
De préférence, les particules d'aérogel de silice hydrophobe susceptibles d'être utilisées dans la présente invention présentent avantageusement une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm.
De préférence, les particules d'aérogel de silice hydrophobe susceptibles d'être utilisées dans la présente invention présentent avantageusement une densité tassée p allant de 0,04 g/cm³ à 0,10 g/cm³, de préférence de 0,05 g/cm³ à 0,08 g/cm³.
De préférence, les particules d'aérogel de silice hydrophobe susceptibles d'être utilisées dans la présente invention présentent avantageusement une surface spécifique par unité de volume (SV) allant de 5 à 60 m²/cm³, de préférence de 10 à 50 m²/cm³ et mieux de 15 à 40 m²/cm³.

Selon un mode de réalisation préféré, les particules d'aérogel de silice hydrophobe selon l'invention présentent une surface spécifique par unité de masse (SM) allant de 500 à 1500 m²/g, de préférence de 600 à 1200 m²/g et mieux de 600 à 800 m²/g, et une taille exprimée en diamètre moyen (D[0,5]) allant de 1 à 30 µm et/ou une capacité d'absorption d'huile mesurée au WET POINT allant de 5 à 18 ml/g de particules, de préférence de 6 à 15 ml/g et mieux de 8 à 12 ml/g.

Selon un autre mode de réalisation préféré, les particules d'aérogel de silice hydrophobe utilisées dans la présente invention présentent une surface spécifique par unité de masse (SM) allant de 600 à 800 m²/g et une taille exprimée en diamètre moyen en volume (D[0,5]) allant de 5 à 20 µm, mieux de 5 à 15 µm.

La surface spécifique par unité de masse peut être déterminée par la méthode d'absorption d'azote appelée méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.
La capacité d'absorption mesurée au WET POINT, et notée Wp, correspond à la quantité d'huile qu'il faut additionner à 100 g de particules pour obtenir une pâte homogène. Elle est mesurée selon la méthode dite de Wet Point ou méthode de détermination de prise d'huile de poudre selon le principe décrit dans la norme NF T 30-022. Elle correspond à la quantité d'huile adsorbée sur la surface disponible de la poudre et/ou absorbée par la poudre par mesure du Wet Point, décrite ci-dessous :
On place une quantité m = 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.
La prise d'huile (capacité d'absorption d'huile) correspond au rapport Vs / m.
Les tailles des particules d'aérogel selon l'invention peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.
Dans le cadre de la présente invention, la densité tassée peut être appréciée selon le protocole suivant, dit protocole de la densité tassée : 40 g de poudre sont versés dans une éprouvette graduée puis l'éprouvette est placée sur un appareil STAV 2003 de chez STAMPF VOLUMETER. L'éprouvette est ensuite soumise à une série de 2500 tassements (cette opération est recommencée jusqu'à ce que la différence de volume entre 2 essais consécutifs soit inférieure à 2 %); puis le volume final Vf de poudre tassée est mesuré directement sur l'éprouvette. La densité tassée est déterminée par le rapport masse(m)/Vf, en l'occurrence 40/Vf (Vf étant exprimé en cm³ et m en g).
La surface spécifique par unité de volume est donnée par la relation : SV = SM x p, où p est la densité tassée exprimée en g/cm³ et SM est la surface spécifique par unité de masse exprimée en m²/g, telles que définies plus haut.

Les particules d'aérogel de silice hydrophobe utilisées selon la présente invention sont de préférence des particules d'aérogel de silice silylée (nom INCI silica silylate). La préparation de particules d'aérogels de silice hydrophobe modifiées en surface par silylation est décrite plus avant dans le document US 7,470,725. On utilisera en particulier des particules d'aérogels de silice hydrophobe modifiée en surface par groupements triméthylsilyles.

A titre de d'aérogels de silice hydrophobe utilisables dans l'invention, on peut citer par exemple l'aérogel commercialisé sous la dénomination VM-2260 (nom INCI Silica silylate), par la société Dow Corning, dont les particules présentent une taille moyenne d'environ 1000 microns et une surface spécifique par unité de masse allant de 600 à 800 m²/g.
On peut également citer les aérogels commercialisés par la société Cabot sous les références AEROGEL TLD 201, AEROGEL OGD 201 et AEROGEL TLD 203, ENOVA AEROGEL MT 1100, ENOVA AEROGEL MT 1200.
On utilisera plus particulièrement l'aérogel commercialisé sous la dénomination VM-2270 (nom INCI Silica silylate), par la société Dow Corning, dont les particules présentent une taille moyenne allant de 5 à 15 microns et une surface spécifique par unité de masse allant de 600 à 800 m²/g.

On peut bien évidemment utiliser un mélange de particules d'aérogel de silice hydrophobe.

Les compositions selon l'invention peuvent comprendre les particules d'aérogel de silice hydrophobe en une quantité comprise entre 0,01 et 20% en poids, de préférence entre 0,05 et 10% en poids, préférentiellement entre 0,1 et 5% en poids par rapport au poids total de la composition.

### Polymères à motif sucre

La composition selon l'invention comprend également au moins un polymère à motif(s) sucre(s). On peut bien évidemment utiliser un mélange de tels polymères.

Préférentiellement, le polymère à motif(s) sucre(s) est un polymère épaississant.
Au sens de la présente invention, on entend par "polymère épaississant" un polymère qui, introduit à 1% en poids dans une solution aqueuse ou hydroalcoolique à 30% d'éthanol, et à pH = 7, permet d'atteindre une viscosité d'au moins 100 cps, de préférence au moins 500 cps, à 25°C et à un taux de cisaillement de 1s⁻¹. Cette viscosité peut être mesurée à l'aide d'un viscosimètre cône/plan (Rhéomètre Haake R600 ou analogue).

Par "motif sucre", on entend un motif issu d'un carbohydrate de formule Cₙ(H₂O)ₙ₋₁ ou (CH₂O)ₙ pouvant être éventuellement modifié par substitution et/ou par oxydation et/ou par déshydratation.
Les motifs sucre susceptibles d'entrer dans la composition des polymères de l'invention sont de préférence issus des sucres suivants : glucose, galactose, arabinose, rhamnose, mannose, xylose, fucose, fructose, anhydrogalactose, acide galacturonique, acide glucuronique, acide mannuronique, galactose sulfate, anhydrogalactose sulfate.

Les polymères à motif(s) sucre(s) selon l'invention peuvent être d'origine naturelle ou synthétique. Ils peuvent être non ioniques, anioniques, amphotères ou cationiques. Les unités de base des polymères à motif sucre de l'invention peuvent être des mono- ou disaccharides.

On peut notamment citer comme polymères susceptibles d'être employés, les gommes natives suivantes, ainsi que leurs dérivés :
a) les exsudats d'arbres ou d'arbustes dont :
   - la gomme arabique (polymère ramifié de galactose, d'arabinose, de rhamnose et d'acide glucuronique)
   - la gomme ghatti (polymère issu d'arabinose, de galactose, de mannose, de xylose et d'acide glucuronique)
   - la gomme karaya (polymère issu d'acide galacturonique, de galactose, de rhamnose et d'acide glucuronique)
   - la gomme tragacanthe (ou adragante) (polymère d'acide galacturonique, de galactose, de fucose, de xylose et d'arabinose)
b) les gommes issues d'algues dont :
   - l'agar (polymère issu de galactose et d'anhydrogalactose)
   - les alginates (polymères d'acide mannuronique et d'acide glucuronique)
   - les carraghénanes et les furcelleranes (polymères de galactose sulfate et d'anhydrogalactose sulfate)
c) les gommes issues de semences ou tubercules dont :
   - la gomme de guar (polymère de mannose et de galactose)
   - la gomme de caroube (polymère de mannose et de galactose)
   - la gomme de fenugrec (polymère de mannose et de galactose)
   - la gomme de tamarin (polymère de galactose, de xylose et de glucose)
   - la gomme de konjac (polymère de glucose et mannose) d) les gommes microbiennes dont :
   - la gomme de xanthane (polymère de glucose, de mannose acétate, de mannose/acide pyruvique et d'acide glucuronique)
   - la gomme de gellane (polymère de glucose partiellement acylé, de rhamnose et d'acide glucuronique)
   - la gomme de scléroglucane (polymère du glucose)
e) les extraits de plantes dont :
   - la cellulose (polymère du glucose)
   - l'amidon (polymère du glucose)
   - l'inuline (polymère de fructose et de glucose).

Ces polymères peuvent être modifiés par voie physique ou chimique. A titre de traitement physique, on peut citer notamment la température. A titre de traitements chimiques, on peut citer les réactions d'estérification, d'étherification, d'amidification, d'oxydation. Ces traitements permettent de conduire à des polymères qui peuvent être non ioniques, anioniques, cationiques ou amphotères.

De préférence ces traitements chimiques ou physiques sont appliqués sur les gommes de guar, les gommes de caroube, les amidons et les celluloses.

Les gommes de guar non ioniques utilisables selon l'invention peuvent être modifiées par des groupements hydroxylakyle en C1-C6. Parmi les groupements hydroxyalkyle, on peut mentionner les groupements hydroxyméthyle, hydroxyéthyle, hydroxypropyle et hydroxybutyle.
Ces gommes de guar sont bien connues de l'état de la technique et peuvent par exemple être préparées en faisant réagir des oxydes d'alcènes correspondants tels que par exemple des oxydes de propylène avec la gomme de guar, de façon à obtenir une gomme de guar modifiée par des groupements hydroxypropyle.
Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.
De telles gommes de guar non ioniques éventuellement modifiées par des groupements hydroxyalkyle sont par exemple vendues sous les dénominations commerciales JAGUAR HP8, JAGUAR HP60 et JAGUAR HP120 par la société RHODIA CHIMIE.

Les gommes de guar modifiées par des groupements cationiques plus particulièrement utilisables selon l'invention sont des gommes de guar comportant des groupements cationiques trialkylammonium. De préférence, 2 à 30% en nombre des fonctions hydroxyle de ces gommes de guar portent des groupements cationiques trialkyammonium. Encore plus préférentiellement, 5 à 20% du nombre des fonctions hydroxyle de ces gommes de guar sont branchés par des groupements cationiques trialkyammonium. Parmi ces groupements trialkylammonium, on peut tout particulièrement citer les groupements triméthylammonium et triéthylammonium. Encore plus préférentiellement, ces groupements représentent de 5 à 20% en poids par rapport au poids total de la gomme de guar modifiée.
Selon l'invention, on peut utiliser des gommes de guar modifiée par du chlorure de 2,3-époxypropyl triméthylammonium.
Ces gommes de guar modifiées par des groupements cationiques sont des produits déjà connus en eux-mêmes et sont par exemple décrits dans les brevets US 3 589 578 et US 4 0131 307. De tels produits sont par ailleurs vendus notamment sous les dénominations commerciales de JAGUAR C13 S, JAGUAR C 15, JAGUAR C 17 par la société RHODIA CHIMIE.

Comme gomme de caroube modifiée, on peut utiliser la gomme de caroube cationique contenant des groupements hydroxypropyltrimmonium telle que le Catinal CLB 200 proposé par la société TOHO.

Les molécules d'amidons utilisés dans la présente invention peuvent avoir comme origine toutes les sources végétales d'amidon, notamment les céréales et les tubercules; plus particulièrement il peut s'agir d'amidons de maïs, de riz, de manioc, d'orge, de pomme de terre, de blé, de sorgho, de pois, d'avoine, de tapioca. On peut également utiliser les hydrolysats des amidons cités ci-dessus. L'amidon est de préférence issu de la pomme de terre.
Les amidons peuvent être modifiés par voie chimique ou physique, notamment par une ou plusieurs des réactions suivantes : prégélatinisation, oxydation, réticulation, estérification, éthérification, amidification, traitements thermiques.
De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyméthyl, octénylsuccinique.

On peut notamment obtenir par réticulation avec des composés phosphorés des phosphates de monoamidon (du type Am-O-PO-(OX)₂), des phosphates de diamidon (du type Am-O-PO-(OX)-O-Am) ou même de triamidon (du type Am-O-PO-(O-Am)₂) ou leurs mélanges; avec Am signifiant amidon et X désignant notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.
Les composés phosphorés peuvent être par exemple du tripolyphosphate de sodium, de l'orthophosphate de sodium, de l'oxychlorure de phosphore ou du trimétaphosphate de sodium.
On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).
Un amidon préféré est un amidon ayant subi au moins une modification voie chimique telle qu'au moins une estérification.

Selon l'invention, on peut aussi utiliser des amidons amphotères, comprenant un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate, et de préférence carboxylique. Les groupements cationiques peuvent être de type amine primaire, secondaire, tertiaire ou quaternaire.
Les amidons amphotères sont notamment choisis parmi les composés de formules suivantes : 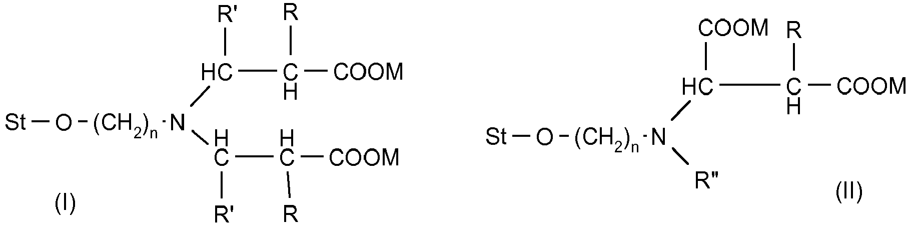 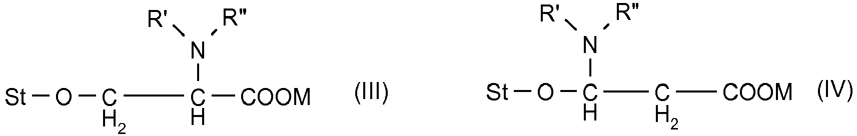 dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH4, un ammonium quaternaire ou une amine organique,
R" représente un atome d'hydrogène ou un radical alkyle en C1-C18.
Ces composés sont notamment décrits dans US5455340 et US4017460.

On utilise particulièrement les amidons de formules (II) ou (III); et préférentiellement les amidons modifiés par de l'acide 2-chloroéthylaminodipropionique, c'est-à-dire les amidons de formule (II) ou (III) dans lesquelles R, R', R" et M représentent un atome d'hydrogène et n est égal à 2. L'amidon amphotère préféré est un chloroéthylamido dipropionate d'amidon.

Les celluloses et dérivés de celluloses peuvent être anioniques, cationiques, amphotères ou non ioniques. Parmi ces dérivés, on distingue les éthers de celluloses, les esters de celluloses et les esters éthers de celluloses.
Parmi les esters de celluloses, on peut citer les esters inorganiques de cellulose (nitrates, sulfates ou phosphates de cellulose), les esters organiques de cellulose (monoacétates, triacétates, amidopropionates, acétatebutyrates, acétatepropionates ou acétatetrimellitates de cellulose) et les esters mixtes organique/inorganique de cellulose tels que les acétatebutyratesulfates et les acétate-propionatesulfates de cellulose.
Parmi les esters éthers de celluloses, on peut citer les phtalates d'hydroxypropyl méthylcellulose et les sulfates d'éthylcellulose.
Parmi les éthers de cellulose non ioniques, on peut citer les alkylcelluloses telles que les méthylcelluloses et les éthylcelluloses (par exemple l'Ethocel standard 100 Premium de DOW CHEMICAL); les hydroxyalkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes hydroxyalkyl-alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutylméthylcelluloses.
Parmi les éthers de cellulose anioniques, on peut citer les carboxyalkylcelluloses et leurs sels. Comme exemple, on peut citer les carboxyméthylcelluloses, les carboxyméthylméthylcelluloses (par exemple Blanose 7M de la société AQUALON) et les carboxyméthylhydroxyéthylcelluloses, ainsi que leurs sels de sodium.
Parmi les éthers de cellulose cationiques, on peut citer les hydroxyéthylcelluloses quaternisées réticulées ou non. L'agent quaternisant peut être notamment le chlorure de diallyldimethylammonium (par exemple Celquat L200 de NATIONAL STARCH). Comme autre éther de cellulose cationique, on peut citer l'hydroxyéthyl cellulose hydroxypropyltriméthylammonium (par exemple Ucare polymer JR 400 d'AMERCHOL).

Parmi les polymères épaississants à motif(s) sucre(s), associatifs, on peut citer les celluloses ou leurs dérivés, modifiés par des groupements comportant au moins une chaîne grasse tels que des groupes alkyls, arylalkyls, alkylaryls ou leurs mélanges où les groupes alkyls sont en C8-C22; les alkylhydroxyéthylcelluloses non-ioniques telles que les produits NATROSOL PLUS GRADE 330 CS et POLYSURF 67 (alkyle en C16) vendus par la société AQUALON; les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C12) et QUATRISOFT LM-X 529-8 (alkyle en C18) vendus par la société AMERCHOL, les produits CRODACEL QM, CRODACEL QL (alkyle en C12) et CRODACEL QS (alkyle en C18) vendus par la société CRODA et le produit SOFTCAT SL 100 vendu par la société AMERCHOL; les nonoxynylhydroxyéthylcelluloses non ioniques tels que le produit AMERCELL HM-1500 vendu par la société AMERCHOL; les alkylcelluloses non-ioniques telles que le produit BERMOCOLL EHM 100 vendu par la société BEROL NOBEL.

Comme polymères à motif(s) sucre(s) dérivés de guar associatifs, on peut citer les hydroxypropylguars modifiés par une chaîne grasse tel que le produit ESAFLOR HM 22 (modifié par une chaîne alkyle en C22) vendu par la société LAMBERTI ; le produit MIRACARE XC 95-3 (modifié par une chaîne alkyle en C14) et le produit RE 205-146 (modifié par une chaîne alkyle en C20) vendus par RHODIA CHIMIE.

Le ou les polymères à motif(s) sucre(s) de l'invention sont choisis de préférence parmi les gommes de guar, les gommes de caroube, les gommes de xanthane, les amidons et les celluloses, dans leur forme modifiée (dérivés) ou non modifiée.

De préférence, les polymères à motif(s) sucre(s) selon l'invention sont non ioniques.

La composition selon l'invention comprend le ou les polymères à motif(s) sucre(s) de préférence en une quantité comprise entre 0,01 et 10% en poids, notamment de 0,05 à 5% en poids, préférentiellement de 0,1 à 1% en poids, voire de 0,1 à 0,5% en poids, par rapport au poids total de la composition.

De préférence le rapport pondéral polymère(s) à motif(s) sucre(s) / particules d'aérogel de silice hydrophobe varie de 0,1 à 10, mieux de 0,2 à 5.

### Autres ingrédients

La composition cosmétique selon l'invention comprend généralement un milieu cosmétiquement acceptable, c'est-à-dire un milieu compatible avec les matières kératiniques telles que la peau du visage ou du corps, les lèvres, les cheveux, les cils, les sourcils et les ongles.

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.
Ces compositions peuvent être conditionnées dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

La composition selon l'invention peut être aqueuse ou anhydre. Elle est de préférence aqueuse et comprend alors de l'eau à une concentration allant de préférence de 5 à 98% en poids, notamment de 20 à 95% en poids, mieux de 50 à 90% en poids, par rapport au poids total de la composition
La composition peut également comprendre un ou plusieurs solvants organiques liquides à 25°C, 1 atm., notamment hydrosolubles, tels que les alcools en C1-C7; on peut notamment citer les monoalcools aliphatiques ou aromatiques en C1-C7, les polyols et les éthers de polyols en C3-C7, qui peuvent donc être employés seuls ou en mélange avec de l'eau. Avantageusement, le solvant organique peut être choisi parmi l'éthanol, l'isopropanol, l'alcool benzylique et leurs mélanges.

La composition selon l'invention peut en outre comprendre au moins un ingrédient cosmétique usuel, différent des composés de l'invention, et notamment choisi parmi les propulseurs; les huiles végétales, minérales, animales ou de synthèse; les corps gras solides et notamment les cires, les esters en C8-C40, les acides en C8-C40; les alcools en C8-C40; les tensioactifs non ioniques, les tensioactifs cationiques, les tensioactifs anioniques, les tensioactifs amphotères, les tensioactifs zwittérioniques; les filtres solaires; les agents hydratants; les agents antipelliculaires; les agents antioxydants; les agents chélatants; les agents réducteurs; les bases d'oxydation, les coupleurs, les agents oxydants, les colorants directs; les agents défrisants tels que les thiols et les hydroxydes alcalins; les agents nacrants et opacifiants; les agents plastifiants ou de coalescence; les hydroxyacides; les pigments; les charges; les silicones et en particulier les polydiméthylsiloxanes (PDMS); les épaississants sans motif(s) sucre(s); les émulsionnants; les polymères conditionneurs ou coiffants; les parfums; les agents d'alcalinisation ou d'acidification; les silanes; les agents de réticulation tels que les polyphénols, les aldéhydes, la DHA. La composition peut bien évidemment comprendre plusieurs ingrédients cosmétiques figurant dans la liste ci-dessus.
Selon leur nature et la destination de la composition, les ingrédients cosmétiques usuels peuvent être présents en des quantités usuelles, aisément déterminables par l'homme du métier, et qui peuvent être comprises, de manière générale, pour chaque ingrédient, entre 0,01 à 80% en poids.

Les huiles peuvent être préférentiellement présentes à raison de 0,01 à 80% en poids, notamment 0,02 à 40% en poids, voire 0,5 à 20% en poids, par rapport au poids total de la composition. Elles peuvent être carbonées. On peut notamment citer les huiles végétales, animales ou minérales, hydrogénées ou non, les huiles synthétiques hydrocarbonées, cycliques ou aliphatiques, linéaires ou ramifiées, saturées ou insaturées, telles que par exemple les polyalpha-oléfines, en particulier les polydécènes et polyisobutènes; les alcools gras liquides tels que l'alcool isostéarylique, l'octyldodécanol, le 2-butyloctanol, le 2-hexyldécanol, le 2-undécylpentadécanol, l'alcool oléique ou l'alcool linoléique; les esters liquides comme le myristate d'isopropyle ou le palmitate d'isopropyle. On peut aussi citer les huiles de silicone, volatiles ou non, organomodifiées ou non, hydrosolubles ou non; les huiles fluorées ou perfluorées; ainsi que leurs mélanges.
La composition peut aussi comprendre un ou plusieurs corps gras solides, et en particulier un ou plusieurs alcools gras, esters gras et/ou acides gras, différents des huiles ci-dessus, ayant 8 à 40 atomes de carbone; ces corps gras solides peuvent préférentiellement être présents à raison de 0,01 à 30% en poids, notamment 0,1 à 20% en poids par rapport au poids total de la composition. On peut notamment citer les alcools gras à chaînes linéaires en C12-C32, notamment en C12-C26, et en particulier l'alcool cétylique, l'alcool stéarylique, l'alcool cétylstéarylique, l'alcool béhénylique. On peut citer également les acides gras à chaînes linéaires ou ramifiées en C16-C40, et notamment l'acide méthyl-18 eicosanoïque, les acides d'huile de coprah ou d'huile de coprah hydrogénée; l'acide stéarique, l'acide laurique, l'acide palmitique et l'acide oléique, l'acide béhénique, et leurs mélanges. De préférence, les acides gras sont non salifiés. On peut encore citer les esters gras à chaînes linéaires, comportant au total entre 8 et 40 atomes de carbone, tels que les myristates, palmitates et stéarates de myristyle, de cétyle, de stéaryle, seuls ou en mélange.
L'homme de métier veillera à choisir les ingrédients entrant dans la composition, ainsi que leurs quantités, de manière à ce qu'ils ne nuisent pas aux propriétés des compositions de la présente invention.

Le pH de la composition, si elle est aqueuse, peut être acide, neutre ou alcalin. De préférence, la composition présente un pH compris entre 2 et 11, notamment 3 à 9, voire 3 à 7.

La composition cosmétique selon l'invention peut se présenter sous la forme d'un produit de soin, de nettoyage et/ou de maquillage de la peau du corps ou du visage, des lèvres, des sourcils, des cils, des ongles et des cheveux, d'un produit solaire ou autobronzant, d'un produit d'hygiène corporelle, d'un produit capillaire, notamment de soin, de nettoyage, de coiffage, de mise en forme, de coloration des cheveux.

Elle trouve notamment une application particulièrement intéressante dans le domaine capillaire, notamment pour le maintien de la coiffure ou la mise en forme des cheveux, ou encore le soin, le traitement cosmétique ou le nettoyage des cheveux. Les compositions capillaires sont de préférence des shampooings, des après-shampooings, des gels de coiffage ou de soin, des lotions ou crèmes de soin, des conditionneurs, des masques, des sérums, des lotions de mise en plis, des lotions pour le brushing, des compositions de fixation et de coiffage telles que les laques ou spray; de lotion restructurante pour cheveux; de lotion ou gel anti-chute, de shampoing antiparasitaire, de lotion ou shampoing antipelliculaire, de shampoing traitant antiséborrhéique. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

La composition selon l'invention trouve une application particulièrement intéressante pour le soin, le traitement et/ou le nettoyage des cheveux et/ou du cuir chevelu, par exemple en shampooing, en après shampooing, en post-traitement d'un shampoing et/ou d'un après-shampoing; ou bien entre deux shampoings.
On peut ainsi par exemple appliquer la composition selon l'invention sur le cuir chevelu et/ou les cheveux, directement après un shampooing, sur cheveux humides ou sur cheveux secs, avec ou sans temps de pause, avec ou sans chaleur, ladite application étant suivie ou pas d'un rinçage et d'un séchage, soit à température ambiante soit avec un sèche-cheveu ou encore avec un fer à lisser (250°C) par exemple.
On peut également appliquer la composition selon l'invention sur le cuir chevelu et/ou les cheveux directement après un après-shampooing, sur cheveux humides ou sur cheveux secs, avec ou sans temps de pause, avec ou sans chaleur, ladite application étant suivie ou pas d'un rinçage et d'un séchage, soit à température ambiante soit avec un sèche-cheveu ou encore avec un fer à lisser (250°C) par exemple.
On peut encore appliquer la composition selon l'invention sur le cuir chevelu et/ou les cheveux entre deux shampooings, sur cheveux humides ou sur cheveux secs.

La composition cosmétique peut être rincée ou non après avoir été appliquée sur les matières kératiniques (cheveux et/ou cuir chevelu).

L'invention a également pour objet un procédé de traitement cosmétique, notamment de maquillage, de soin, de nettoyage, de coloration, de mise en forme, des matières kératiniques, notamment de la peau du corps ou du visage, des lèvres, des ongles, des cheveux et/ou des cils, comprenant l'application sur lesdites matières d'une composition cosmétique selon l'invention.

Il s'agit en particulier d'un procédé de traitement capillaire, pour le soin, le traitement cosmétique et/ou le nettoyage des cheveux et/ou du cuir chevelu, et en particulier permettant de retarder voire d'éviter le regraissage des cheveux et/ou du cuir chevelu, et ainsi d'espacer les shampoings.

L'invention est illustrée plus en détails dans les exemples suivants.

### Exemples

On prépare les compositions de soin capillaire suivantes (% en poids) :

| | Composition A |
|---|---|
| SILICA SILYLATE (nom INCI) | 0,5% |
| Hydroxypropylméthylcellulose (HPMC) METHOCEL F 4 M de DOW CHEMICAL | 0,3% |
| Eau | Qsp 100% |
| | |
| | Composition B |
| SILICA SILYLATE (nom INCI) | 0,5% |
| Hydroxyéthylcellulose NATROSOL 250 HHR PC de ASHLAND | 0,3% |
| Eau | Qsp 100% |
| | |
| | Composition C |
| SILICA SILYLATE (nom INCI) | 0,5% |
| Gomme de xanthane RHODICARE XC de RHODIA | 0,3% |
| Eau | Qsp 100% |

Le SILICA SILYLATE employé est le produit commercialisé sous le nom DOW CORNING VM-2270 AEROGEL FINE PARTICLES par Dow Corning.

Les compositions sont faciles à appliquer sur les cheveux et le cuir chevelu; elles permettent de réduire le regraissage des cheveux.

## Revendications

1. Composition cosmétique comprenant :
- des particules d'aérogel de silice hydrophobe,
- au moins un polymère à motif(s) sucre(s); et
- de l'eau à une concentration allant de 20 à 98% en poids, par rapport au poids total de la composition.

2. Composition selon la revendication 1, dans laquelle les particules d'aérogel de silice hydrophobe présentent une surface spécifique par unité de masse (SM) allant de 500 à 1500 m²/g, de préférence de 600 à 1200 m²/g et mieux de 600 à 800 m²/g,

3. Composition selon l'une quelconque des revendications précédentes, dans laquelle les particules d'aérogel de silice hydrophobe présentent une capacité d'absorption d'huile mesurée au WET POINT allant de 5 à 18 ml/g de particules, de préférence de 6 à 15 ml/g et mieux de 8 à 12 ml/g.

4. Composition selon l'une quelconque des revendications précédentes, dans laquelle les particules d'aérogel de silice hydrophobe présentent une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm.

5. Composition selon l'une quelconque des revendications précédentes, dans laquelle les particules d'aérogel de silice hydrophobe présentent une densité tassée p allant de 0,04 g/cm³ à 0,10 g/cm³, de préférence de 0,05 g/cm³ à 0,08 g/cm³.

6. Composition selon l'une quelconque des revendications précédentes, dans laquelle les particules d'aérogel de silice hydrophobe présentent une surface spécifique par unité de volume (SV) allant de 5 à 60 m²/cm³, de préférence de 10 à 50 m²/cm³ et mieux de 15 à 40 m²/cm³.

7. Composition selon l'une quelconque des revendications précédentes, dans laquelle les particules d'aérogel de silice hydrophobe sont des particules d'aérogel de silice silylée et notamment des particules d'aérogels de silice hydrophobe modifiée en surface par groupements triméthylsilyles.

8. Composition selon l'une quelconque des revendications précédentes, dans laquelle les particules d'aérogel de silice hydrophobe sont présentes en une quantité comprise entre 0,01 et 20% en poids, de préférence entre 0,05 et 10% en poids, préférentiellement entre 0,1 et 5% en poids par rapport au poids total de la composition.

9. Composition selon l'une quelconque des revendications précédentes, dans laquelle les motifs sucre susceptibles d'entrer dans la composition des polymères sont issus des sucres suivants : glucose, galactose, arabinose, rhamnose, mannose, xylose, fucose, fructose, anhydrogalactose, acide galacturonique, acide glucuronique, acide mannuronique, galactose sulfate, anhydrogalactose sulfate.

10. Composition selon l'une quelconque des revendications précédentes, dans laquelle les polymères à motif(s) sucre(s) sont non ioniques, anioniques, amphotères ou cationiques.

11. Composition selon l'une quelconque des revendications précédentes, dans laquelle les polymères à motif(s) sucre(s) sont choisis parmi les gommes de guar, les gommes de caroube, les gommes de xanthane, les amidons et les celluloses, modifiés ou non modifiés.

12. Composition selon l'une quelconque des revendications précédentes, dans laquelle les polymères à motif(s) sucre(s) est présent en une quantité comprise entre 0,01 et 10% en poids, notamment de 0,05 à 5% en poids, préférentiellement de 0,1 à 1% en poids, voire de 0,1 à 0,5% en poids, par rapport au poids total de la composition.

13. Composition selon l'une quelconque des revendications précédentes, dans laquelle le rapport pondéral polymère(s) à motif(s) sucre(s) / particules d'aérogel de silice hydrophobe varie de 0,1 à 10, mieux de 0,2 à 5.

14. Composition selon l'une quelconque des revendications précédentes, comprenant de l'eau à une concentration allant de 20 à 95% en poids, mieux de 50 à 90% en poids, par rapport au poids total de la composition.

15. Composition selon l'une quelconque des revendications précédentes, se présentant sous la forme d'un produit capillaire, notamment pour le soin, le traitement cosmétique ou le nettoyage des cheveux et/ou du cuir chevelu.

16. Procédé de traitement cosmétique, notamment de maquillage, de soin, de nettoyage, de coloration, de mise en forme, des matières kératiniques, notamment de la peau du corps ou du visage, des lèvres, des ongles, des cheveux et/ou des cils, comprenant l'application sur lesdites matières d'une composition cosmétique selon l'une des revendications 1 à 15.

17. Procédé selon la revendication 16, de traitement capillaire, pour le soin, le traitement cosmétique et/ou le nettoyage des cheveux et/ou du cuir chevelu, en particulier permettant de retarder voire d'éviter le regraissage des cheveux et/ou du cuir chevelu.

## Patentansprüche

1. Kosmetikzusammensetzung, umfassend:
- Aerogelpartikel aus hydrophobem Siliziumdioxid,
- mindestens ein Polymer mit Zuckermotiv bzw.
Zuckermotiven und
- Wasser in einer Konzentration von 20 bis 98 Gew.-%, bezogen auf das Gesamtgewicht der Zusammensetzung.

2. Zusammensetzung nach Anspruch 1, wobei die Aerogelpartikel aus hydrophobem Siliziumdioxid eine spezifische Oberfläche pro Masseneinheit (SM) von 500 bis 1500 m²/g, vorzugsweise von 600 bis 1200 m²/g und noch besser von 600 bis 800 m²/g aufweisen.

3. Zusammensetzung nach einem der vorhergehenden Ansprüche, wobei die Aerogelpartikel aus hydrophobem Siliziumdioxid eine mittels WET POINT gemessene Ölabsorptionskapazität von 5 bis 18 ml/g Partikel, vorzugsweise von 6 bis 15 ml/g und noch besser von 8 bis 12 ml/g aufweisen.

4. Zusammensetzung nach einem der vorhergehenden Ansprüche, wobei die Aerogelpartikel aus hydrophobem Siliziumdioxid eine Größe, ausgedrückt als mittlerer Durchmesser (D[0,5]), von unter 1500 µm und vorzugsweise von 1 bis 30 µm, vorzugsweise von 5 bis 25 µm, noch besser von 5 bis 20 µm und noch viel besser von 5 bis 15 µm aufweisen.

5. Zusammensetzung nach einem der vorhergehenden Ansprüche, wobei die Aerogelpartikel aus hydrophobem Siliziumdioxid eine Klopfdichte p von 0,04 g/cm³ bis 0,10 g/cm³, vorzugsweise von 0,05 g/cm³ bis 0,08 g/cm³ aufweisen.

6. Zusammensetzung nach einem der vorhergehenden Ansprüche, wobei die Aerogelpartikel aus hydrophobem Siliziumdioxid eine spezifische Oberfläche pro Volumeneinheit (SV) von 5 bis 60 m²/cm³, vorzugsweise von 10 bis 50 m²/cm³ und noch besser von 15 bis 40 m²/cm³ aufweisen.

7. Zusammensetzung nach einem der vorhergehenden Ansprüche, wobei es sich bei den Aerogelpartikeln aus hydrophobem Siliziumdioxid um silylierte Siliziumdioxid-Aerogelpartikel und insbesondere um Aerogelpartikel aus hydrophobem Siliziumdioxid, die mit Trimethylsilylgruppen oberflächenmodifiziert sind, handelt.

8. Zusammensetzung nach einem der vorhergehenden Ansprüche, wobei die Aerogelpartikel aus hydrophobem Siliziumdioxid in einer Menge zwischen 0,01 und 20 Gew.-%, vorzugsweise zwischen 0,05 und 10 Gew.-%, bevorzugt zwischen 0,1 und 5 Gew.-%, bezogen auf das Gesamtgewicht der Zusammensetzung, vorliegen.

9. Zusammensetzung nach einem der vorhergehenden Ansprüche, wobei die Zuckermotive, die in der Zusammensetzung der Polymere vorhanden sein können, von den folgenden Zuckern stammen: Glucose, Galactose, Arabinose, Rhamnose, Mannose, Xylose, Fucose, Fructose, Anhydrogalactose, Galacturonsäure, Glucuronsäure, Mannuronsäure, Galactosesulfat, Anhydrogalactosesulfat.

10. Zusammensetzung nach einem der vorhergehenden Ansprüche, wobei die Polymere mit Zuckermotiv bzw. Zuckermotiven nichtionisch, anionisch, amphoter oder kationisch sind.

11. Zusammensetzung nach einem der vorhergehenden Ansprüche, wobei die Polymere mit Zuckermotiv bzw. Zuckermotiven aus Guargummen, Johannesbrotkernmehlen, Xanthangummen, Stärken und Cellulosen, die modifiziert oder unmodifiziert sind, ausgewählt sind.

12. Zusammensetzung nach einem der vorhergehenden Ansprüche, wobei die Polymere mit Zuckermotiv bzw. Zuckermotiven in einer Menge zwischen 0,01 und 10 Gew.-%, insbesondere von 0,05 bis 5 Gew.-%, vorzugsweise von 0,1 bis 1 Gew.-%, sogar von 0,1 bis 0,5 Gew.-%, bezogen auf das Gesamtgewicht der Zusammensetzung, vorhanden sind.

13. Zusammensetzung nach einem der vorhergehenden Ansprüche, wobei das Gewichtsverhältnis von Polymer(en) mit Zuckermotiv bzw. Zuckermotiven/Aerogelpartikeln aus hydrophobem Siliziumdioxid von 0,1 bis 10, besser von 0,2 bis 5 variiert.

14. Zusammensetzung nach einem der vorhergehenden Ansprüche, die Wasser in einer Konzentration von 20 bis 95 Gew.-%, besser von 50 bis 90 Gew.-%, bezogen auf das Gesamtgewicht der Zusammensetzung, umfasst.

15. Zusammensetzung nach einem der vorhergehenden Ansprüche, die in Form eines Haarpflegemittels, insbesondere für die Pflege, die kosmetische Behandlung oder das Waschen der Haare und/oder der Kopfhaut, vorliegt.

16. Verfahren zur kosmetischen Behandlung, insbesondere zum Schminken, Pflegen, Waschen, Färben, Formen von Keratinsubstanzen, insbesondere der Haut des Körpers oder des Gesichts, der Lippen, der Nägel, der Haare und/oder der Wimpern, das die Applikation einer Kosmetikzusammensetzung nach einem der Ansprüche 1 bis 15 auf diese Substanzen umfasst.

17. Haarbehandlungsverfahren nach Anspruch 16 zur Pflege, kosmetischen Behandlung und/oder zum Waschen der Haare und/oder der Kopfhaut, das es insbesondere gestattet, das erneute Fettigwerden der Haare und/oder der Kopfhaut zu verzögern oder sogar zu vermeiden.

## Claims

1. Cosmetic composition comprising:
- hydrophobic silica aerogel particles,
- at least one polymer bearing sugar unit(s); and
- water at a concentration ranging from 20% to 98% by weight, relative to the total weight of the composition.

2. Composition according to Claim 1, wherein the hydrophobic silica aerogel particles have a specific surface area per unit of mass (S_{M}) ranging from 500 to 1500 m²/g, preferably from 600 to 1200 m²/g and better still from 600 to 800 m²/g.

3. Composition according to either one of the preceding claims, wherein the hydrophobic silica aerogel particles have an oil absorption capacity, measured at the wet point, ranging from 5 to 18 ml/g, preferably from 6 to 15 ml/g and better still from 8 to 12 ml/g of particles.

4. Composition according to any one of the preceding claims, wherein the hydrophobic silica aerogel particles have a size, expressed as the mean diameter (D[0.5]), of less than 1500 µm, and preferably ranging from 1 to 30 µm, preferably from 5 to 25 µm, better still from 5 to 20 µm and even better still from 5 to 15 µm.

5. Composition according to any one of the preceding claims, wherein the hydrophobic silica aerogel particles have a tapped density ρ ranging from 0.04 g/cm³ to 0.10 g/cm³ and preferably from 0.05 g/cm³ to 0.08 g/cm³.

6. Composition according to any one of the preceding claims, wherein the hydrophobic silica aerogel particles have a specific surface area per unit of volume (S_{V}) ranging from 5 to 60 m²/cm³, preferably from 10 to 50 m²/cm³ and better still from 15 to 40 m²/cm³.

7. Composition according to any one of the preceding claims, wherein the hydrophobic silica aerogel particles are silylated silica aerogel particles and in particular aerogel particles of hydrophobic silica surface-modified with trimethylsilyl groups.

8. Composition according to any one of the preceding claims, wherein the hydrophobic silica aerogel particles are present in an amount of between 0.01% and 20% by weight, preferably between 0.05% and 10% by weight and preferentially between 0.1% and 5% by weight, relative to the total weight of the composition.

9. Composition according to any one of the preceding claims, wherein the sugar units that may be included in the composition of the polymers are derived from the following sugars: glucose, galactose, arabinose, rhamnose, mannose, xylose, fucose, fructose, anhydrogalactose, galacturonic acid, glucuronic acid, mannuronic acid, galactose sulfate, anhydrogalactose sulfate.

10. Composition according to any one of the preceding claims, wherein the polymers bearing sugar unit(s) are nonionic, anionic, amphoteric or cationic.

11. Composition according to any one of the preceding claims, wherein the polymers bearing sugar unit(s) are chosen from modified or unmodified guar gums, locust bean gums, xanthan gums, starches and celluloses.

12. Composition according to any one of the preceding claims, wherein the polymers bearing sugar unit(s) are present in an amount of between 0.01% and 10% by weight, especially from 0.05% to 5% by weight, preferentially from 0.1% to 1% by weight, or even from 0.1% to 0.5% by weight, relative to the total weight of the composition.

13. Composition according to any one of the preceding claims, wherein the polymer(s) bearing sugar unit(s)/hydrophobic silica aerogel particles weight ratio varies from 0.1 to 10, better still from 0.2 to 5.

14. Composition according to any one of the preceding claims, comprising water at a concentration ranging from 20% to 95% by weight and better still from 50% to 90% by weight, relative to the total weight of the composition.

15. Composition according to any one of the preceding claims, being in the form of a haircare product, especially for the care, cosmetic treatment or cleansing of the hair and/or of the scalp.

16. Cosmetic treatment method, especially for making up, caring for, cleansing, coloring or shaping keratin materials, especially bodily or facial skin, the lips, the nails, the hair and/or the eyelashes, comprising the application to said materials of a cosmetic composition according to one of Claims 1 to 15.

17. Hair treatment method according to Claim 16, for the care, cosmetic treatment and/or cleansing of the hair and/or of the scalp, in particular that makes it possible to delay or even prevent regreasing of the hair and/or of the scalp.
